(19) Europäisches Patentamt
European Patent Office
Office européen des brevets

(11) EP 1 773 355 B1

(12) **EUROPEAN PATENT SPECIFICATION**

(45) Date of publication and mention of the grant of the patent:
**25.06.2014 Bulletin 2014/26**

(21) Application number: **05789867.8**

(22) Date of filing: **20.06.2005**

(51) Int Cl.:
***A61K 31/70*** *(2006.01)* ***C07H 19/00*** *(2006.01)*

(86) International application number:
**PCT/US2005/021684**

(87) International publication number:
**WO 2006/012078 (02.02.2006 Gazette 2006/05)**

(54) **NUCLEOSIDE ARYL PHOSPHORAMIDATES FOR THE TREATMENT OF RNA-DEPENDENT RNA VIRAL INFECTION**

NUKLEOSID-ARYL-PHOSPORAMIDATE ZUR BEHANDLUNG VON RNA-ABHÄNGIGER RNA-VIRALINFEKTION

PHOSPHORAMIDATES D'ARYLE NUCLEOSIDIQUES POUR LE TRAITEMENT D'INFECTION VIRALE ARN DEPENDANTE DE L'ARN

(84) Designated Contracting States:
**AT BE BG CH CY CZ DE DK EE ES FI FR GB GR HU IE IS IT LI LT LU MC NL PL PT RO SE SI SK TR**
Designated Extension States:
**LV**

(30) Priority: **24.06.2004 US 582667 P**
**18.10.2004 US 619746 P**

(43) Date of publication of application:
**18.04.2007 Bulletin 2007/16**

(73) Proprietor: **Merck Sharp & Dohme Corp.**
**Rahway, NJ 07065-0907 (US)**

(72) Inventors:
- **MACCOSS, Malcolm**
  **Rahway, NJ 07065-0907 (US)**
- **OLSEN, David, B.**
  **Rahway, NJ 07065-0907 (US)**

(74) Representative: **Man, Jocelyn**
**Merck & Co., Inc.**
**European Patent Department**
**Hertford Road**
**Hoddesdon**
**Hertfordshire EN11 9BU (GB)**

(56) References cited:
**WO-A-00/00501**
**WO-A-02/057287**
**WO-A-02/057425**
**WO-A-03/000713**
**US-B1- 6 475 985**
**US-B2- 6 638 919**
**US-B2- 6 660 721**

- **C. MCGUIGAN ET AL: "Intracellular Delivery of Bioactive AZT Nucleotides by Aryl Phosphate Derivatives of AZT" JOURNAL OF MEDICINAL CHEMISTRY, vol. 36, 1993, pages 1048-1052, XP002109804**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

FIELD OF THE INVENTION

The present invention is concerned with nucleoside aryl phosphoramidates, their synthesis, and their use as precursors to inhibitors of RNA-dependent RNA viral polymerase. The compounds of the present invention are precursors to inhibitors of RNA-dependent RNA viral replication and are therefore useful for the treatment of RNA-dependent RNA viral infection. They are particularly useful as precursors to inhibitors of hepatitis C virus (HCV) NS5B polymerase, as precursors to inhibitors of HCV replication, and for the treatment of hepatitis C infection.

BACKGROUND OF THE INVENTION

Hepatitis C virus (HCV) infection is a major health problem that leads to chronic liver disease, such as cirrhosis and hepatocellular carcinoma, in a substantial number of infected individuals, estimated to be 2-15% of the world's population. There are an estimated 4.5 million infected people in the United States alone, according to the U.S. Center for Disease Control. According to the World Health Organization, there are more than 200 million infected individuals worldwide, with at least 3 to 4 million people being infected each year. Once infected, about 20% of people clear the virus, but the rest harbor HCV the rest of their lives. Ten to twenty percent of chronically infected individuals eventually develop liver-destroying cirrhosis or cancer. The viral disease is transmitted parenterally by contaminated blood and blood products, contaminated needles, or sexually and vertically from infected mothers or carrier mothers to their off-spring. Current treatments for HCV infection, which are restricted to immunotherapy with recombinant interferon-$\alpha$ alone or in combination with the nucleoside analog ribavirin, are of limited clinical benefit. Moreover, there is no established vaccine for HCV. Consequently, there is an urgent need for improved therapeutic agents that effectively combat chronic HCV infection. The state of the art in the treatment of HCV infection has been reviewed, and reference is made to the following publications: B. Dymock, et al., "Novel approaches to the treatment of hepatitis C virus infection," Antiviral Chemistry & Chemotherapy, 11: 79-96 (2000); H. Rosen, et al., "Hepatitis C virus: current understanding and prospects for future therapies," Molecular Medicine Today, 5: 393-399 (1999); D. Moradpour, et al., "Current and evolving therapies for hepatitis C," European J. Gastroenterol. Hepatol., 11: 1189-1202 (1999); R. Bartenschlager, "Candidate Targets for Hepatitis C Virus-Specific Antiviral Therapy," Intervirology, 40: 378-393 (1997); G.M. Lauer and B.D. Walker, "Hepatitis C Virus Infection," N. Engl. J. Med., 345: 41-52 (2001); B.W. Dymock, "Emerging therapies for hepatitis C virus infection," Emerging Drugs, 6: 13-42 (2001); and C. Crabb, "Hard-Won Advances Spark Excitement about Hepatitis C," Science: 506-507 (2001); the contents of all of which are incorporated by reference herein in their entirety.

Different approaches to HCV therapy have been taken, which include the inhibition of viral serine proteinase (NS3 protease), helicase, and RNA-dependent RNA polymerase (NS5B), and the development of a vaccine.

The HCV virion is an enveloped positive-strand RNA virus with a single oligoribonucleotide genomic sequence of about 9600 bases which encodes a polyprotein of about 3,010 amino acids. The protein products of the HCV gene consist of the structural proteins C, E1, and E2, and the non-structural proteins NS2, NS3, NS4A and NS4B, and NS5A and NS5B. The nonstructural (NS) proteins are believed to provide the catalytic machinery for viral replication. The NS3 protease releases NS5B, the RNA-dependent RNA polymerase from the polyprotein chain. HCV NS5B polymerase is required for the synthesis of a double-stranded RNA from a single-stranded viral RNA that serves as a template in the replication cycle of HCV. NS5B polymerase is therefore considered to be an essential component in the HCV replication complex [see K. Ishi, et al., "Expression of Hepatitis C Virus NS5B Protein: Characterization of Its RNA Polymerase Activity and RNA Binding," Hepatology, 29: 1227-1235 (1999) and V. Lohmann, et al., "Biochemical and Kinetic Analyses of NS5B RNA-Dependent RNA Polymerase of the Hepatitis C Virus," Virology, 249: 108-118 (1998)]. Inhibition of HCV NS5B polymerase prevents formation of the double-stranded HCV RNA and therefore constitutes an attractive approach to the development of HCV-specific antiviral therapies.

The development of inhibitors of HCV NSSB polymerase with potential for the treatment of HCV infection has been reviewed in M.P. Walker et al., "Promising candidates for the treatment of chronic hepatitis C," Expert Opin. Invest. Drugs, 12: 1269-1280 (2003) and in P. Hoffmann et al., "Recent patents on experimental therapy for hepatitis C virus infection (1999-2002)," Expert Opin. Ther. Patents," 13: 1707-1723 (2003). The activity of purine ribonucleosides against HCV polymerase was reported by A.E. Eldrup et al., "Structure-Activity Relationship of Purine Ribonucleosides for Inhibition of HCV RNA-Dependent RNA Polymerase," J. Med. Chem., 47: 2283-2295 (2004). There is a continuing need for structurally diverse nucleoside derivatives as inhibitors of HCV polymerase as therapeutic approaches for HCV therapy.

WO 02/057425 discloses nucleoside derivatives as inhibitors of RNA-dependent RNA viral polymerase, in particular as inhibitors of hepatitis C virus (HCV) NS5B polymerase.

It has now been found that nucleoside aryl phosphoramidates of the present invention are precursors to potent inhibitors of RNA-dependent RNA viral replication and in particular HCV replication. The phosphoramidates are converted

*in vivo* into their nucleoside 5'-phosphate (nucleotide) derivatives which are converted into the corresponding nucleoside 5'-triphosphate derivatives which are inhibitors of RNA-dependent RNA viral polymerase and in particular HCV NS5B polymerase. The instant nucleoside phosphoramidates are useful to treat RNA-dependent RNA viral infection and in particular HCV infection.

It it therefore an object of the present invention to provide nucleoside aryl phosphorarmidates which are useful as precursors to inhibitors of RNA-rdependent RNA viral polymerase and in particular as precursors to inhibitors of HCV NS5B polymerase.

It is another object the present invention to provide nucleoside aryl phosphoramidates which are useful as precursors to inhibit the replication of an RNA-dependeht RNA virus and in particular as precursor to inhibitors of the replication of hepatitis C virus.

It is another object of the present invention to provide nucleoside aryl phosphoramidates which are useful in the treatment of RNA-depend RNA viral infection and in a particular in the treatment of HGV infection

It is another object of the present invention to provide pharmaceutical compositions comprising the nucleoside aryl phosphoramidates of the present invention in association with a pharmaceutically acceptable carrier.

It is another object of the present invention to provide pharmaceutical compositions comprising the nucleoside aryl phosphoramidates of the present invention for use as precursors to inhibitors of RNA-dependent RNA-viral polymerase and in particular as precursors to inhibitors of HCV NS5B polymerase.

It is another object of the present invention to provide pharmaceutical compositions comprising the nucleoside aryl phosphoramidates of the present invention for use as precursors to inhibitors of RNA-dependent RNA-viral polymerase and in particular as precursors to inhibitors of HCV replication.

It is another object of the present invention to provide pharmaceutical compositions comprising the nucleoside aryl phosphoramidates of the present invention for use in the treatment of RNA-dependent RNA viral infection and in particular in the treatment of HCV infection.

It is another object of the present invention to provide pharmaceutical compositions comprising the nucleoside aryl phosphoramidates of the present invention in combination with other agents active against RNA-dependent RNA virus and in particular against HCV.

Also disclosed are methods for the inhibition of RNA-dependent RNA viral polymerase and in particular for the inhibition of HCV NS5B polymerase.

Also disclosed are methods for the inhibition of RNA-dependent RNA viral replication and in particular for the inhibition of HCV replication.

Also disclosed are methods for the treatment of RNA-dependent RNA viral infection and in particular for the treatment of HCV infection.

Also disclosed are methods for the treatment of RNA-dependent RNA viral infection in combination with other agents active against RNA-dependent RNA virus and in particular for the treatment of HCV infection in combination with other agents active against HGV.

It is another object of the present invention to provide nucleoside aryl phosphoramidates and their pharmaceutical for use as medicament for the inhibition of RNA-dependent RNA viral replication and/or the treatment of RNA-dependent RNA viral infection and in particular for the inhibition of HCV replication and/or the treatment of HCV infection.

It is another object of the present invention to provide for the use of the nucleoside aryl phosphoramidates of the present invention and their pharmaceutical compositions for the manufacture of a medicament for the inhibition of RNA-dependent RNA viral replication and/or the treatment of RNA-dependent RNA viral infection and in particular for the inhibition of HCV replication and/or the treatment of HCV infection

These and other objects will become readily apparent from the detailed description which follows:

## SUMMARY OF THE INVENTION

The present invention relates to compounds of structural formula I of the indicated stereochemical configuration:

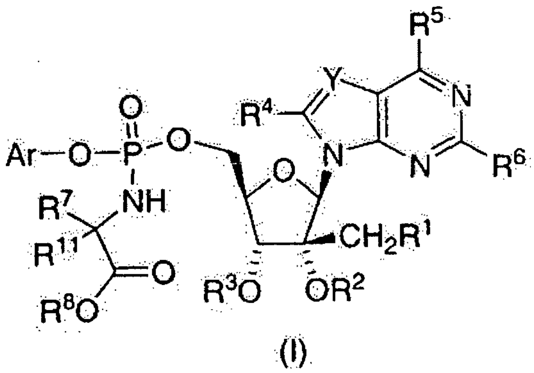

(I)

or pharmaceutically acceptable salt thereof; wherein
Y is N;
Ar is phenyl unsubstituted or substituted with one to three substituents independently selected from the group consisting of halogen, $C_{1-4}$ alkyl, $C_{1-4}$ alkoxy, $C_{1-4}$ alkylthio, cyano, nitro, amino, carboxy, trifluoromethyl, $C_{1-4}$ alkylamino, di($C_{1-4}$ alkyl)amino, $C_{1-4}$alkylcarbonyl, $C_{1-4}$alkylcarbonyloxy, and $C_{1-4}$ alkyloxycarbonyl;
$R^1$ is selected from the group consisting of hydrogen, fluoro, azido, amino, hydroxy, $C_{1-3}$ alkoxy, mercapto, and $C_{1-3}$ alkylthio;
$R^2$ and $R^3$ are each independently selected from the group consisting of hydrogen, methyl, $C_{1-16}$ alkylcarbonyl, $C_{2-18}$ alkenylcarbonyl, $C_{1-10}$ alkylcarboxycarbonyl, $C_{3-6}$ cycloalkylcarbonyl, and $C_{3-6}$ cycloalkyloxycarbonyl;
$R^4$ is hydrogen, halogen, methyl, azido, or amino;
$R^5$ and $R^6$ are each independently-selected from the group consisting of hydrogen, hydroxy, halogen, $C_{1-4}$ alkoxy, amino, $C_{1-4}$ alkylamino, di($C_{1-4}$alkyl)amino, $C_{3-6}$ cycloalkylamino, di($C_{3-6}$ cycloalkyl)amino, benzylamino, dibenzylamino, or $C_{4-6}$ heterocycloalkyl, wherein alkyl, cycloalkyl, benzyl, and heterocycloalkyl are unsubstituted or substituted with one to five groups independently selected from halogen hydroxy; amino, $C_{1-4}$alkyl, and $C_{1-4}$alkoxy;
$R^7$ is hydrogen, $C_{1-5}$ alkyl, phenyl or benzyl;
wherein alkyl is unsubstituted or substituted with one substituent selected from the group consisting of hydroxy, methoxy, amino, carboxy, carbamoyl, guanidino, mercapto, methylthio, 1*H*-imidazolyl, and 1*H*-indol-3-yl; and wherein phenyl and benzyl are unsusbtituted or substituted with one to two substituents independently selected from the group consisting of halogen, hydroxy and methoxy;
$R^8$ is hydrogen, $C_{1-6}$ alkyl, $C_{3-6}$ cycaoalkyl, phenyl, or benzyl;
wherein alkyl and cycloalkyl are unsubstituted or substituted with one to three substituents independently selected from halogen, hydroxy, carboxy, $C_{1-4}$alkoxy; and wherein phenyl and benzyl are unsubstituted or substituted with one to three substituents independently selected from halogen, hydroxy, cyano, $C_{1-4}$alkoxy, and trifluoromethyl; and
$R^{11}$ is, hydrogen or methyl.

The compounds of formula I are useful as precursors to inhibitors of RNA-dependent. RNA viral polymerase and in particular of HCV NS5B polymerase. They are also precursors to inhibitors of RNA-dependent RNA viral replication and in particular of HCV replication and are useful for the treatment of RNA-dependent RNA viral infection and in particular for the treatment HCV infection.

Without limitation as to their mechanism of action, the aryl phosphoramidates of the present invention act as prodrugs of the corresponding nucleoside, 5'-monophosphates. Endogenous kinase enzymes convert the 5'-monophosphates into their 5'-triphosphate derivatives which are the inhibitors of the RNA-dePendent RNA viral polymerase. Thus, the aryl phosphoramidates may provide for more efficient target cell penetration than the nucleoside itself, may be less susceptible to metabolic degradation, and may have the ability to target to specific such as the liver, resulting in a wider therapeutic index allowing for lowering the overall dose of the antiviral agent.

Also encompassed within the present invention are pharmaceutical compositions containing the compounds alone or in combination with other agents active against RNA-dependent RNA virus and in particular against HCV as well methods for the inhibition of RNA-dependent RNA viral replication and for the treatment of RNA-dependent RNA viral infection.

DETAILED DESCRIPTION OF THE INVENTION

The present invention relates to compounds of structural Formula I of the indicated stereochemical configuration:

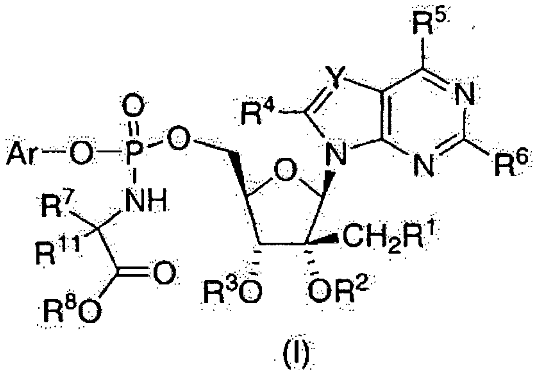

(I)

or a pharmaceutically acceptable salt thereof; wherein

Y is N;

Ar is phenyl unsubstituted or substituted with one to three substituents independently selected from the group consisting of halogen, $C_{1-4}$ alkyl, $C_{1-4}$ alkoxy, $C_{1-4}$ alkylthio, cyano, nitro, amino, carboxy, trifluoromethyl, $C_{1-4}$ alkylamino, di($C_{1-4}$alkyl)amino, $C_{1-4}$ alkylcarbonyl, $C_{1-4}$ alkylcarbonyloxy, and $C_{1-4}$ alkyloxycarbonyl;

$R^1$ is selected from the group consisting of hydrogen, fluoro, azido, amino, hydroxy, $C_{1-3}$ alkoxy, mercapto, and $C_{1-3}$ alkylthio;

$R^2$ and $R^3$ are each independently selected from the group consisting of hydrogen, methyl, $C_{1-16}$. alkylcarbonyl, $C_{2-18}$ alkenylcarbonyl, $C_{1-10}$ alkyloxycarbonyl, $C_{3-6}$ cycloalkylcarbonyl, and $C_{3-6}$ cycloalkyloxycarbonyl;

$R^4$ is hydrogen, halogen, methyl, azido, or amino;

$R^5$ and $R^6$ are each independently selected from the group consisting of hydrogen, hydroxy, halogen, $C_{1-4}$ alkoxy, amino, $C_{1-4}$ alkylamino, di($C_{1-4}$ alkyl)amino, $C_{3-6}$ cycloalkylamino; di($C_{3-6}$ cycloalkyl)amino, benzylamino, dibenzylamino, or $C_{4-6}$ heterocycloalkyl, wherein alkyl, cycloalkyl, benzyl, and heterocycloalkyl are unsubstituted or substituted with one to five groups independently selected from halogen, hydroxy, amino, $C_{1-4}$ alkyl, and $C_{1-4}$ alkoxy,

$R^7$ is hydrogen, $C_{1-5}$ alkyl, phenyl or benzyl;

wherein alkyl is unsubstituted or substituted with one substituent selected from the group consisting of hydroxy, methoxy,amino, carboxy, carbamoyl, guanidino, mercapto, methylthio, 1*H*-imidazolyl, and 1*H*-indol-3-yl; and wherein phenyl and benzyl are unsubstituted or substituted with one to two substituents independently selected from the group consisting of halogen, hydroxy, and methoxy;

$R^8$ is hydrogen, $C_{1-6}$ alkyl, alkyl, $C_{3-6}$ cycloalkyl, phenyl, or benzyl;

wherein alkyl and cycloalkyl are unsubstituted or substituted with one to three substituents independently selected from halogen, hydroxy, carboxy, $C_{1-4}$ alkoxy, and wherein phenyl and benzyl are unsubstituted or substituted with one to three substituents independently selected from halogen, hydroxy, cyano, $C_{1-4}$ alkoxy, and trifluoromethyl; and

$R^{11}$ is hydrogen or methyl.

The compounds of formula I are useful as precursors to inhibitors of RNA-dependent. NA, viral polymerase. They are also precursors to inhibitors of RNA dependent RNA viral replication and are useful for treatment of RNA-dependent RNA viral infection.

In one embodiment of the compounds of the present invention, Y is N, $R^1$ is hydrogen or fluoro, and $R^2$ and $R^3$ are hydrogen. In a class of this embodiment, $R^4$ is hydrogen.

In a second embodiment of the compounds of the present invention, Ar is unsubstituted phenyl.

In a third embodiment of the compounds of the present invention, $R^{11}$ is hydrogen and $R^7$ is selected from the group consisting of hydrogen, methyl, ethyl, n-propyl; isopropyl, isobutyl, 2-methyl-1-propyl,hydroxymethyl, mercaptomethyl, carboxymethyl, carbamoylmethyl, 1-hydroxyethyl, 2-carboxyethyl, 2-carbamoylethyl, 2-methylthioethyl, 4-amino-1-butyl, 3-amino-2-propyl, 3-guanidino-1-propyl, 1*H*-imidazol-4-ylmethyl, phenyl, 4-hydroxybenzyl, and 1*H*-indol-3-ylmethyl. In a class of this embodiment, $R^7$ is methyl or benzyl.

In a fourth embodiment of the compounds of the present invention, $R^8$ is $C_{1-6}$ alkyl, cyclohexyl, phenyl or benzyl. In a class of this embodiment, $R^8$ is methyl.

In a fifth embodiment of the compounds of the present invention, Ar is unsubstituted phenyl, $R^7$ is methyl or benzyl , $R^8$ is methyl, and $R^{11}$ is hydrogen.

Illustrative but nonlimiting examples of compounds of the present invention of structural formula I which are useful as precursors to inhibitors of RNA-dependent RNA viral polymerase are the following:

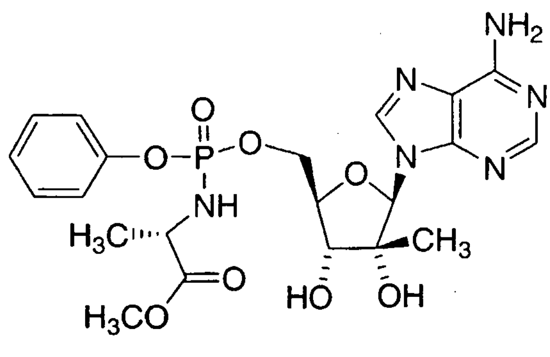

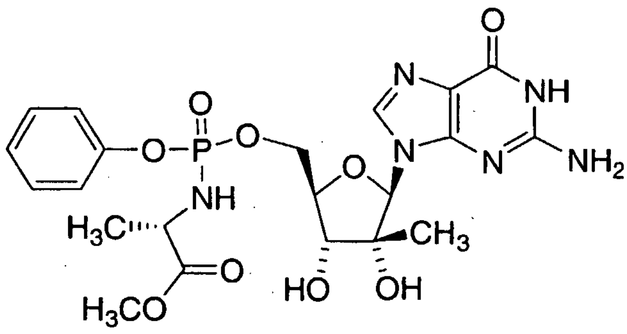

or a pharmaceutically acceptable salt thereof.

Also disclosed is, the nucleoside aryl phosphoramidates of the present invention are useful as precursors to inhibitors of positive-sense single stranded RNA-dependent RNA viral polymerase, inhibitors of positive sense single-stranded RNA-dependent RNA viral replication, and/or for the treatment of positive-sense single-stranded RNA-dependent RNA viral infection. In a class of this embodiment, the positive-sense single-stranded RNA-dependent RNA virus is a *Flaviviridae* virus or *a Picornaviridae* virus. In a subclass of this class, the *Picornaviridae* virus is a rhinovirus, a poliovirus, or a hepatitis A virus In a second subclass of this class, the *Flaviridae* virus is is selected from the group consisting of hepatitis C virus, yellow fever virus, dengue virus, West Nile virus, Japanese-encephalitis virus, Banzi virus; and bovine viral diarrhea virus (BVDV). In a subclass of this subclass, the *Flaviviridae* virus is hepatitis C virus.

Also disclosed is a method for inhibiting RNA-dependent RNA viral polymerase, a method for inhibiting RNA-dependent RNA viral replication, and/or a method for treating RNA-dependent RNA viral infection in a mammal in need thereof comprising administering to the mammal a therapeutically effective amount of a compound of structural formula I.

Also disclosed is, the RNA-dependent RNA viral polymerase is a positive-sense single-stranded RNA-dependent RNA viral polymerase. In a class of this embodiment, the positive-sense single-stranded RNA-dependent RNA viral polymerase is a *Flaviviridae* viral polymerase or a *Picornaviridae* viral polymerase. In a subclass of this class, the *Picornaviridae* viral polymerase is rhinovirus polymerase, poliovirus polymerase, or hepatitis A virus polymerase. In a second subclass of this class, the *Flaviviridae* viral polymerase is selected from the group consisting of hepatitis C virus polymerase, yellow fever virus polymerase, dengue virus polymerase, West Nile virus polymerase, Japanese encephalitis virus polymerase, Banzi virus polymerase, and bovine viral diarrhea virus (BVDV) polymerase. In a subclass of this subclass, the *Flaviviridae* viral polymerase is hepatitis C virus polymerase.

Also disclosed is, the RNA-dependent RNA viral replication is a positive-sense single-stranded RNA-dependent RNA viral replication. In a class of this embodiment, the positive-sense single-stranded RNA-dependent RNA viral replication is *Flaviviridae* viral replication or *Picornaviridae* viral replication. In a subclass of this class, the *Picornaviridae* viral replication is rhinovirus replication, poliovirus replication, or hepatitis A virus replication. In a second subclass of this class, the *Flaviviridae* viral replication is selected from the group consisting of hepatitis C virus replication, yellow fever virus replication, dengue virus replication, West Nile virus replication, Japanese encephalitis virus replication, Banzi virus replication, and bovine viral diarrhea virus replication. In a subclass of this subclass, the *Flaviviridae* viral replication is hepatitis C virus replication.

Also disclosed is the RNA-dependent RNA viral infection is a positive-sense single-stranded RNA-dependent viral infection. In a class of this embodiment, the positive-sense single-stranded RNA-dependent RNA viral infection is *Flaviviridae* viral infection or *Picornaviridae* viral infection. In a subclass of this class, the *Picornaviridae* viral infection is rhinovirus infection, poliovirus infection, or hepatitis A virus infection. In a second subclass of this class, the *Flaviviridae* viral infection is selected from the group consisting of hepatitis C virus infection, yellow fever virus infection, dengue

virus infection, West Nile virus infection, Japanese encephalitis virus infection, Banzi virus infection, and bovine viral diarrhea virus infection. In a subclass of this subclass, the *Flaviviridae* viral infection is hepatitis C virus infection.

Throughout the instant application, the following terms have the indicated meanings:

The alkyl groups specified above are intended to include those alkyl groups of the designated length in either a straight or branched configuration. Exemplary of such alkyl groups are methyl, ethyl, propyl, isopropyl, butyl, sec-butyl, tertiary butyl, pentyl, isopentyl, hexyl, isohexyl, and the like.

The term "alkenyl" shall mean straight or branched chain alkenes of two to six total carbon atoms, or any number within this range (e.g., ethenyl, propenyl, butenyl, pentenyl, etc.).

The term "alkynyl" shall mean straight or branched chain alkynes of two to six total carbon atoms, or any number within this range (e.g., ethynyl, propynyl, butynyl, pentynyl, etc.).

The term "cycloalkyl" shall mean cyclic rings of alkanes of three to eight total carbon atoms, or any number within this range (i.e., cyclopropyl, cyclobutyl, cyclopentyl, cyclohexyl, cycloheptyl, or cyclooctyl).

The term "cycloheteroalkyl" is intended to include non-aromatic heterocycles containing one or two heteroatoms selected from nitrogen, oxygen and sulfur. Examples of 4-6-membered cycloheteroalkyl include azetidinyl, pyrrolidinyl, piperidinyl, morpholinyl, thiamorpholinyl, imidazolidinyl, tetrahydrofuranyl, tetrahydropyranyl, tetrahydrothiophenyl, piperazinyl, and the like.

The term "alkoxy" refers to straight or branched chain alkoxides of the number of carbon atoms specified (e.g., $C_{1-4}$ alkoxy), or any number within this range [i.e., methoxy (MeO-), ethoxy, isopropoxy, etc.].

The term "alkylthio" refers to straight or branched chain alkylsulfides of the number of carbon atoms specified (e.g., $C_{1-4}$ alkylthio), or any number within this range [i.e., methylthio (MeS-), ethylthio, isopropylthio, etc.].

The term "alkylamino" refers to straight or branched alkylamines of the number of carbon atoms specified (e.g., $C_{1-4}$ alkylamino), or any number within this range [i.e., methylamino, ethylamino, isopropylamino, t-butylamino, etc.].

The term "alkylsulfonyl" refers to straight or branched chain alkylsulfones of the number of carbon atoms specified (e.g., $C_{1-6}$ alkylsulfonyl), or any number within this range [i.e., methylsulfonyl ($MeSO_2$-), ethylsulfonyl, isopropylsulfonyl, etc.].

The term "alkyloxycarbonyl" refers to straight or branched chain esters of a carboxylic acid derivative of the present invention of the number of carbon atoms specified (e.g., $C_{1-4}$ alkyloxycarbonyl), or any number within this range [i.e., methyloxycarbonyl (MeOCO-), ethyloxycarbonyl, or butyloxycarbonyl].

The term "halogen" is intended to include the halogen atoms fluorine, chlorine, bromine and iodine.

The term "phosphoryl" refers to $-P(O)(OH)_2$.

The term "diphosphoryl" refers to the radical having the structure:

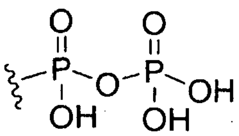

.

The term "triphosphoryl" refers to the radical having the structure:

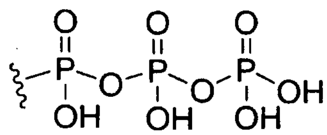

.

The term "substituted" shall be deemed to include multiple degrees of substitution by a named substituent. Where multiple substituent moieties are disclosed or claimed, the substituted compound can be independently substituted by one or more of the disclosed or claimed substituent moieties, singly or plurally.

The term "5'-triphosphate" refers to a triphosphoric acid ester derivative of the 5'-hydroxyl group of a nucleoside compound of the present invention having the following general structural formula II:

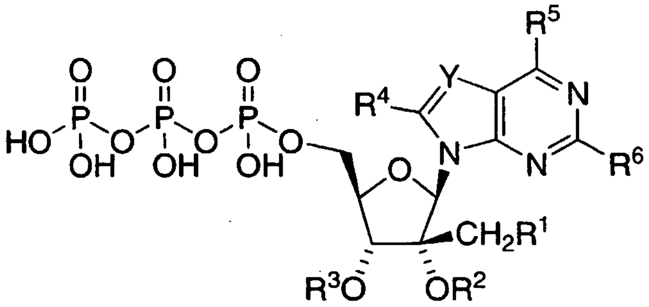

(II)

wherein Y and $R^1$-$R^6$ are as defined above.

The term "composition", as in "pharmaceutical composition," is intended to encompass a product comprising the active ingredient(s) and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

The terms "administration of' and "administering a" compound should be understood to mean providing a compound of the invention or a prodrug of a compound of the invention to the individual in need.

Also disclosed is a method of inhibiting HCV NS5B polymerase, inhibiting HCV replication, or treating HCV infection with a compound of the present invention in combination with one or more agents useful for treating HCV infection. Such agents active against HCV include, but are not limited to, ribavirin, levovirin, viramidine, thymosin alpha-1, interferon-β, interferon-α, pegylated interferon-α (peginterferon-α), a combination of interferon-α and ribavirin, a combination of peginterferon-α and ribavirin, a combination of interferon-α and levovirin, and a combination of peginterferon-α and levovirin. Interferon-α includes, but is not limited to, recombinant interferon-α2a (such as Roferon interferon available from Hoffmann-LaRoche, Nutley, NJ), pegylated interferon-α2a (Pegasys™), interferon-α2b (such as Intron-A interferon available from Schering Corp., Kenilworth, NJ), pegylated interferon-α2b (PegIntron™), a recombinant consensus interferon (such as interferon alphacon-1), and a purified interferon-α product. Amgen's recombinant consensus interferon has the brand name Infergen®. Levovirin is the L-enantiomer of ribavirin which has shown immunomodulatory activity similar to ribavirin. Viramidine represents an analog of ribavirin disclosed in WO 01/60379 (assigned to ICN Pharmaceuticals). In accordance with this method of the present invention, the individual components of the combination can be administered separately at different times during the course of therapy or concurrently in divided or single combination forms. The instant invention is therefore to be understood as embracing all such regimes of simultaneous or alternating treatment, and the term "administering" is to be interpreted accordingly. It will be understood that the scope of combinations of the compounds of this invention with other agents useful for treating HCV infection includes in principle any combination with any pharmaceutical composition for treating HCV infection. When a compound of the present invention or a pharmaceutically acceptable salt thereof is used in combination with a second therapeutic agent active against HCV, the dose of each compound may be either the same as or different from the dose when the compound is used alone.

For the treatment of HCV infection, the compounds of the present invention may also be administered in combination with an agent that is an inhibitor of HCV NS3 serine protease. HCV NS3 serine protease is an essential viral enzyme and has been described to be an excellent target for inhibition of HCV replication. Both substrate and non-substrate based inhibitors of HCV NS3 protease inhibitors are disclosed in WO 98/22496, WO 98/46630, WO 99/07733, WO 99/07734, WO 99/38888, WO 99/50230, WO 99/64442, WO 00/09543, WO 00/59929, GB-2337262, WO 02/48116, WO 02/48172, and U.S. Patent No. 6,323,180. HCV NS3 protease as a target for the development of inhibitors of HCV replication and for the treatment of HCV infection is discussed in B.W. Dymock, "Emerging therapies for hepatitis C virus infection," Emerging Drugs, 6: 13-42 (2001).

Ribavirin, levovirin, and viramidine may exert their anti-HCV effects by modulating intracellular pools of guanine nucleotides via inhibition of the intracellular enzyme inosine monophosphate dehydrogenase (IMPDH). IMPDH is the rate-limiting enzyme on the biosynthetic route in *de novo* guanine nucleotide biosynthesis. Ribavirin is readily phosphorylated intracellularly and the monophosphate derivative is an inhibitor of IMPDH. Thus, inhibition of IMPDH represents another useful target for the discovery of inhibitors of HCV replication. Therefore, the compounds of the present invention may also be administered in combination with an inhibitor of IMPDH, such as VX-497, which is disclosed in WO 97/41211 and WO 01/00622 (assigned to Vertex); another IMPDH inhibitor, such as that disclosed in WO 00/25780 (assigned to Bristol-Myers Squibb); or mycophenolate mofetil [see A.C. Allison and E.M. Eugui, Agents Action, 44 (Suppl.): 165 (1993)].

For the treatment of HCV infection, the compounds of the present invention may also be administered in combination with the antiviral agent amantadine (1-aminoadamantane) [for a comprehensive description of this agent, see J. Kirschbaum, Anal. Profiles Drug Subs. 12: 1-36 (1983)].

The compounds of the present invention may also be combined for the treatment of HCV infection with antiviral 2'-*C*-branched ribonucleosides disclosed in R. E. Harry-O'kuru, et al., J. Org. Chem., 62: 1754-1759 (1997); M. S. Wolfe, et al., Tetrahedron Lett., 36: 7611-7614 (1995); U.S. Patent No. 3,480,613 (Nov. 25, 1969); International Publication Number WO 01/90121 (29 November 2001); International Publication Number WO 01/92282 (6 December 2001); and International Publication Number WO 02/32920 (25 April 2002); and International Publication Number WO 04/002999 (8 January 2004); and International Publication Number WO 04/003000 (8 January 2004); and International Publication Number WO 04/002422 (8 January 2004). Such 2'-C-branched ribonucleosides include, but are not limited to, 2'-C-methyl-cytidine, 2'-C-methyl-uridine, 2'-C-methyl-adenosine, 2'-C-methyl-guanosine, and 9-(2-C-methyl-β-D-ribofuranosyl)-2,6-diaminopurine, and the corresponding amino acid ester of the ribose C-2', C-3', and C-5' hydroxyls and the corresponding optionally substituted cyclic 1,3-propanediol esters of the 5'-phosphate derivatives.

The compounds of the present invention may also be combined for the treatment of HCV infection with other nucleosides having anti-HCV properties, such as those disclosed in WO 02/51425 (4 July 2002), assigned to Mitsubishi Pharma Corp.; WO 01/79246, WO 02/32920, and WO 02/48165 (20 June 2002), assigned to Pharmasset, Ltd.; WO 01/68663 (20 September 2001), assigned to ICN Pharmaceuticals; WO 99/43691 (2 Sept. 1999); WO 02/18404 (7 March 2002), assigned to Hoffmann-LaRoche; U.S. 2002/0019363 (14 Feb. 2002); WO 02/100415 (19 Dec. 2002); WO 03/026589 (3 Apr. 2003); WO 03/026675 (3 Apr. 2003); WO 03/093290 (13 Nov. 2003): US 2003/0236216 (25 Dec. 2003); US 2004/0006007 (8 Jan. 2004); WO 04/011478 (5 Feb. 2004); WO 04/013300 (12 Feb. 2004); US 2004/0063658 (1 Apr. 2004); and WO 04/028481 (8 Apr. 2004).

The compounds of the present invention may also be combined for the treatment of HCV infection with non-nucleoside inhibitors of HCV polymerase such as those disclosed in WO 01/77091 (18 Oct. 2001), assigned to Tularik, Inc.; WO 01/47883 (5 July 2001), assigned to Japan Tobacco, Inc.; WO 02/04425 (17 January 2002), assigned to Boehringer Ingelheim; WO 02/06246 (24 Jan. 2002), assigned to Istituto di Ricerche di Biologia Moleculare P. Angeletti S.P.A.; and WO 02/20497 (3 March 2002).

By "pharmaceutically acceptable" is meant that the carrier, diluent, or excipient must be compatible with the other ingredients of the formulation and not deleterious to the recipient thereof.

Also included within the present invention are pharmaceutical compositions comprising the nucleoside aryl phosphoramidates of the present invention in association with a pharmaceutically acceptable carrier. Another example of the invention is a pharmaceutical composition made by combining any of the compounds described above and a pharmaceutically acceptable carrier. Another illustration of the invention is a process for making a pharmaceutical composition comprising combining any of the compounds described above and a pharmaceutically acceptable carrier.

Also included within the present invention are pharmaceutical compositions useful for inhibiting RNA-dependent RNA viral polymerase in particular HCV NS5B polymerase comprising an effective amount of a compound of the present invention and a pharmaceutically acceptable carrier. Pharmaceutical compositions useful for treating RNA-dependent RNA viral infection in particular HCV infection are also encompassed by the present invention. Also disclosed is a method of inhibiting RNA-dependent RNA viral polymerase in particular HCV NS5B polymerase and a method of treating RNA-dependent viral replication and in particular HCV replication. Additionally, the present invention is directed to a pharmaceutical composition comprising a therapeutically effective amount of a compound of the present invention in combination with a therapeutically effective amount of another agent active against RNA-dependent RNA virus and in particular against HCV. Agents active against HCV include, but are not limited to, ribavirin, levovirin, viramidine, thymosin alpha-1, an inhibitor of HCV NS3 serine protease, interferon-α, pegylated interferon-α (peginterferon-α), a combination of interferon-α and ribavirin, a combination of peginterferon-α and ribavirin, a combination of interferon-α and levovirin, and a combination of peginterferon-α and levovirin. Interferon-α includes, but is not limited to, recombinant interferon-α2a (such as Roferon interferon available from Hoffmann-LaRoche, Nutley, NJ), interferon-α2b (such as Intron-A interferon available from Schering Corp., Kenilworth, NJ), a consensus interferon, and a purified interferon-α product. For a discussion of ribavirin and its activity against HCV, see J.O. Saunders and S.A. Raybuck, "Inosine Monophosphate Dehydrogenase: Consideration of Structure, Kinetics, and Therapeutic Potential," Ann. Rep. Med. Chem., 35: 201-210 (2000).

Another aspect of the present invention provides for the use of the nucleoside aryl phosphoramidates and their pharmaceutical compositions for the manufacture of a medicament for the inhibition of RNA-dependent RNA HCV replication, and/or the treatment of RNA-dependent RNA HCV infection. Yet a further aspect of the present invention provides for the nucleoside aryl phosphoramidates and their pharmaceutical compositions for use as a medicament for the inhibition of RNA-dependent RNA HCV replication, and/or for the treatment of RNA-dependent RNA HCV infection.

The pharmaceutical compositions of the present invention comprise a compound of structural formula I as an active ingredient or a pharmaceutically acceptable salt thereof, and may also contain a pharmaceutically acceptable carrier and optionally other therapeutic ingredients.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of structural formula I can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of structural formula I may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

Any suitable route of administration may be employed for providing a mammal, especially a human with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of structural formula I are administered orally.

For oral administration to humans, the dosage range is 0.01 to 1000 mg/kg body weight in divided doses. In one embodiment the dosage range is 0.1 to 100 mg/kg body weight in divided doses. In another embodiment the dosage range is 0.5 to 20 mg/kg body weight in divided doses. For oral administration, the compositions are preferably provided in the form of tablets or capsules containing 1.0 to 1000 milligrams of the active ingredient, particularly, 1, 5, 10, 15, 20, 25, 50, 75, 100, 150, 200, 250, 300, 400, 500, 600, 750, 800, 900, and 1000 milligrams of the active ingredient for the symptomatic adjustment of the dosage to the patient to be treated.

The effective dosage of active ingredient employed may vary depending on the particular compound employed, the mode of administration, the condition being treated and the severity of the condition being treated. Such dosage may be ascertained readily by a person skilled in the art. This dosage regimen may be adjusted to provide the optimal therapeutic response.

The compounds of the present invention contain one or more asymmetric centers and can thus occur as racemates and racemic mixtures, single enantiomers, diastereomeric mixtures and individual diastereomers. When $R^{11}$ is hydrogen and $R^7$ in the amino acyl residue attached to the phosphorous atom in structural formula I is other than hydrogen in the formula

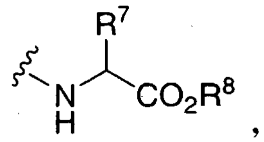

the amino acid residue contains an asymmetric center and is intended to include the individual *R*- and *S*-stereoisomers as well as *RS*-stereoisomeric mixtures. In one embodiment, the stereochemistry at the stereogenic carbon corresponds to that of an *S*-amino acid, that is, the naturally occurring alpha-amino acid stereochemistry.

The tetrasubstituted phosphorous in compounds of structural formula I constitutes another asymmetric center, and the compounds of the present invention are intended to encompass both stereochemical configurations at the phosphorous atom.

The present invention is meant to comprehend nucleoside aryl phosphoramidates having the β-D stereochemical configuration for the five-membered furanose ring as depicted in the structural formula below, that is, nucleoside aryl phosphoramidates in which the substituents at C-1 and C-4 of the five-membered furanose ring have the β-stereochemical configuration ("up" orientation as denoted by a bold line).

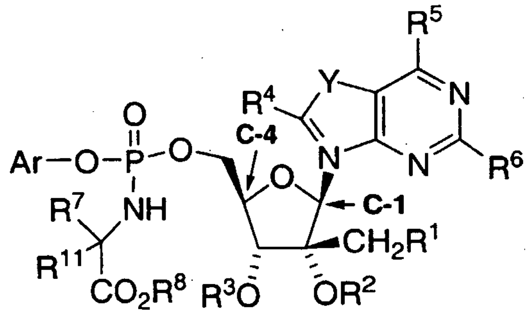

β-D-

Some of the compounds described herein contain olefinic double bonds, and unless specified otherwise, are meant to include both E and Z geometric isomers.

Some of the compounds described herein may exist as tautomers such as keto-enol tautomers. The individual tautomers as well as mixtures thereof are encompassed with compounds of structural formula I. Example of keto-enol tautomers which are intended to be encompassed within the compounds of the present invention are illustrated below:

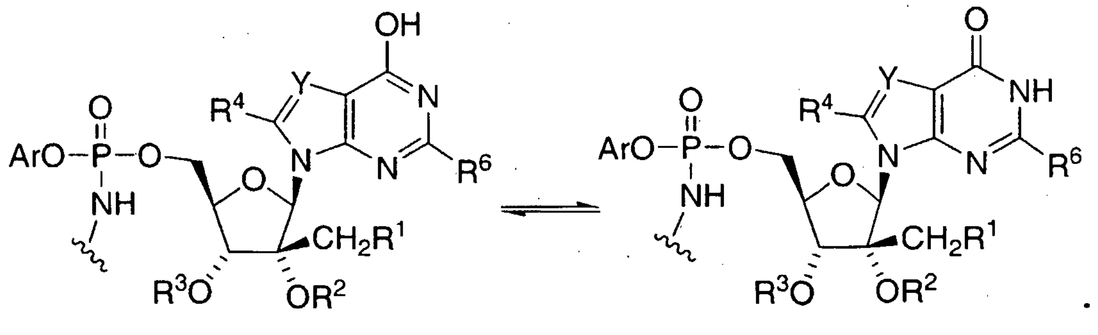

Compounds of structural formula I may be separated into their individual diastereoisomers by, for example, fractional crystallization from a suitable solvent, for example methanol or ethyl acetate or a mixture thereof, or via chiral chromatography using an optically active stationary phase.

Alternatively, any stereoisomer of a compound of the structural formula I may be obtained by stereospecific synthesis using optically pure starting materials or reagents of known configuration.

The compounds of the present invention may be administered in the form of a pharmaceutically acceptable salt. The term "pharmaceutically acceptable salt" refers to salts prepared from pharmaceutically acceptable non-toxic bases or acids including inorganic or organic bases and inorganic or organic acids. Salts of basic compounds encompassed within the term "pharmaceutically acceptable salt" refer to non-toxic salts of the compounds of this invention which are generally prepared by reacting the free base with a suitable organic or inorganic acid. Representative salts

of basic compounds of the present invention include, but are not limited to, the following: acetate, benzenesulfonate, benzoate, bicarbonate, bisulfate, bitartrate, borate, bromide, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methymitrate, methylsulfate, mucate, napsylate, nitrate, N-methylglucamine ammonium salt, oleate, oxalate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate. Furthermore, where the compounds of the invention carry an acidic moiety, suitable pharmaceutically acceptable salts thereof include, but are not limited to, salts derived from inorganic bases including aluminum, ammonium, calcium, copper, ferric, ferrous, lithium, magnesium, manganic, mangamous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, cyclic amines, and basic ion-exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

Also, in the case of a carboxylic acid (-COOH) or hydroxyl group being present in the compounds of the present invention, pharmaceutically acceptable prodrug esters of carboxylic acid derivatives, such as methyl, ethyl, or pivaloyloxymethyl esters or prodrug acyl derivatives of the ribose C-2', C-3', and C-5' hydroxyls, such as *O*-acetyl, *O*-pivaloyl, *O*-benzoyl and *O*-aminoacyl, can be employed. Included are those esters and acyl groups known in the art for modifying the bioavailability, tissue distribution, solubility, and hydrolysis characteristics for use as sustained-release or prodrug formulations. The contemplated derivatives are readily convertible *in vivo* into the required compound. Thus, in the methods of treatment of the present invention, the terms "administering" and "administration" is meant to encompass the treatment of the viral infections described with a compound specifically disclosed or with a compound which may not be specifically disclosed, but which converts to the specified compound *in vivo* after administration to the mammal, including a human patient. Conventional procedures for the selection and preparation of suitable prodrug derivatives are described, for example, in "Design of Prodrugs," ed. H. Bundgaard, Elsevier, 1985.

<u>Preparation of the Aryl Phosphoramidates of the Invention:</u>

The preparation of the nucleoside intermediates for the phosphorylation reaction is described in international patent publications WO 02/057287 (25 July 2002) and WO 02/057425 (25 July 2002). The aryl phosphorochloridates for the phosphorylation reactions were prepared according to the methods described in U.S. Patent No. 6,455,513. The phosphorylation reactions to generate the aryl phosphoroamidates of the present invention were carried out following the methods described in U.S. Patent No. 6,455,513 and C. McGuigan et al., J. Med. Chem., 36: 1048 (1993).

The Examples below provide illustrations of the conditions used for the preparation of the compounds of the present invention. These Examples are not intended to be limitations on the scope of the instant invention in any way, and they should not be so construed. Those skilled in the art of nucleoside and nucleotide synthesis will readily appreciate that known variations of the conditions and processes of the following preparative procedures can be used to prepare these and other compounds of the present invention. All temperatures are degrees Celsius unless otherwise noted.

<u>EXAMPLES 1 AND 2 (REFERENCE)</u>

<u>2'-*C*-Methyladenosine 5'-[phenyl methoxy-(*S*)-alaninylphosphate]</u>

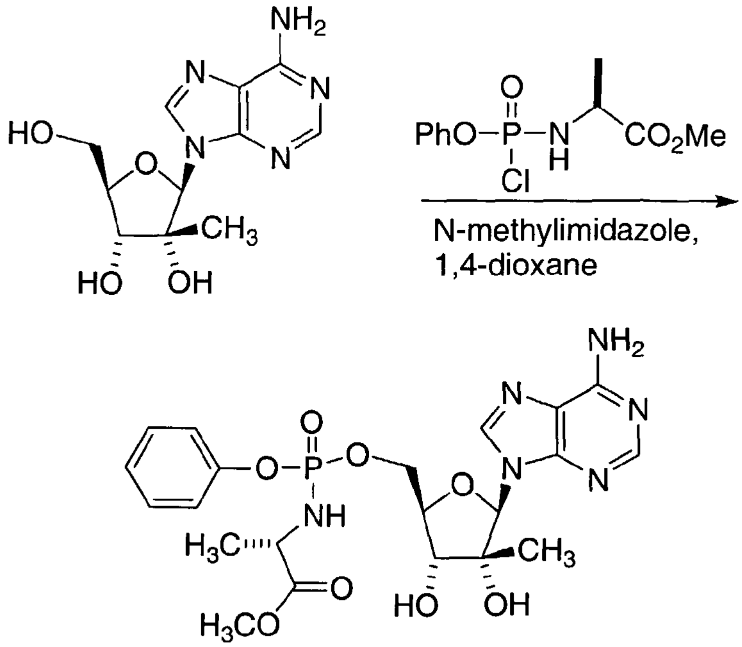


A solution of 2'-*C*-methyladenosine (500 mg), phenyl methoxy-(*S*)-alaninyl phosphorochloridate [1.3 g, prepared according to J. Med. Chem., 36: 1048 (1993)], *N*-methylimidazole (0.8 mL) and 1,4-dioxane (10 mL) was stirred 18 h at ambient temperature. The reaction mixture was concentrated, taken up into saturated aqueous sodium bicarbonate solution and extracted three times with chloroform. The chloroform extracts were dried over anhydrous magnesium sulfate, filtered and concentrated to give a tan solid. The desired product was purified by chromatography on silica gel using 10% methanol/methylene chloride as eluent and then lyophilized to yield a colorless solid obtained as a mixture of diastereomers at the phosphorous atom. The diastereomers were separated using reverse phase liquid chromatography (Kromasil C8, 4.6 x 250 mm, gradient 20%-50% acetonitrile in aqueous 0.1% trifluoroacetic acid over 15 min, 1.5 mL/min) to yield each diastereomer as a colorless solid. Mass spectrum: m/z = 523 for each isomer.

EXAMPLES 3 AND 4

2'-*C*-Methylguanosine 5'-[phenyl methoxy-(*S*)-alaninylphosphate]

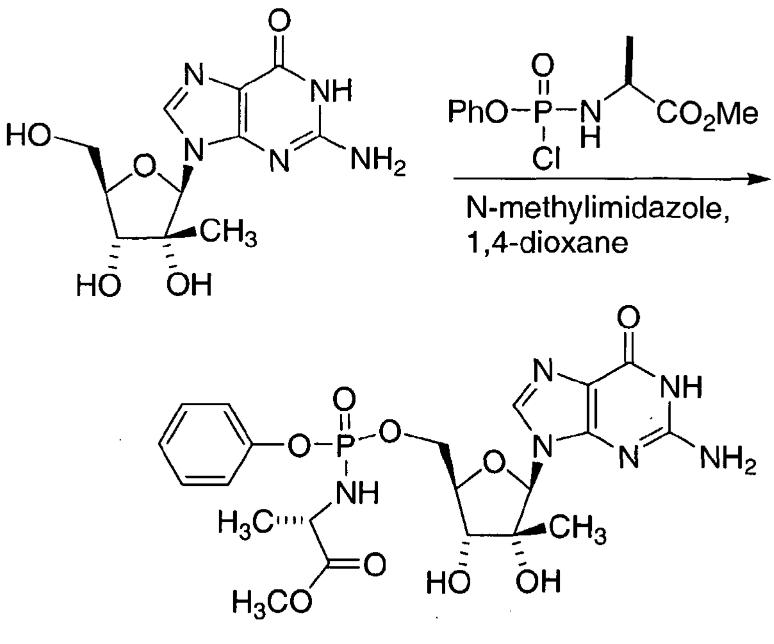

A solution of 2'-*C*-methylguanosine (40 mg), 1,4-dioxane (2 mL), *N*-methylimidazole (70 μL) and the phosphorochloridate (73 mg) was stirred at ambient temperature overnight. The mixture was concentrated to remove the dioxane and partitioned between saturated aqueous sodium bicarbonate solution and chloroform. The desired product remained in the aqueous fraction. The aqueous solution of the diastereomeric mixture was subjected to reverse phase liquid chromatography (Kromasil C8, 4.6 x 250 mm, gradient 20%-50% acetonitrile in aq 0.1% trifluoroacetic acid over 15 min, 1.5mL/min) to yield each diastereomer as a colorless solid. Mass spectrum: m/z = 539 for each isomer.

BIOLOGICAL ASSAYS

The assays employed to measure the inhibition of HCV NS5B polymerase and HCV replication are described below.

The effectiveness of the compounds of the present invention as inhibitors of HCV NS5B RNA-dependent RNA polymerase (RdRp) was measured in the following assay.

A. Assay for Inhibition of HCV NS5B Polymerase:

This assay was used to measure the ability of the nucleoside aryl phosphoramidates of the present invention to inhibit the enzymatic activity of the RNA-dependent RNA polymerase (NS5B) of the hepatitis C virus (HCV) on a heteromeric RNA template.

Procedure:

Assay Buffer Conditions: (50 μL -total/reaction)

20 mM Tris, pH 7.5
50 μM EDTA
5 mM DTT
2 mM $MgCl_2$
80 mM KCl
0.4 U/μL RNAsin (Promega, stock is 40 units/μL)
0.75 μg t500 (a 500-nt RNA made using T7 runoff transcription with a sequence from the NS2/3 region of the hepatitis C genome)
1.6 μg purified hepatitis C NS5B (form with 21 amino acids C-terminally truncated) 1 μM A,C,U,GTP (Nucleoside triphosphate mix)
[alpha-$^{32}$P]-GTP or [alpha-$^{35}$P]-GTP

The compounds were tested at various concentrations up to 100 μM final concentration.

An appropriate volume of reaction buffer was made including enzyme and template t500. Nucleoside derivatives of the present invention were pipetted into the wells of a 96-well plate. A mixture of nucleoside triphosphates (NTP's), including the radiolabeled GTP, was made and pipetted into the wells of a 96-well plate. The reaction was initiated by addition of the enzyme-template reaction solution and allowed to proceed at room temperature for 1-2 h.

The reaction was quenched by addition of 20 μL 0.5M EDTA, pH 8.0. Blank reactions in which the quench solution was added to the NTPs prior to the addition of the reaction buffer were included.

50 μL of the quenched reaction were spotted onto DE81 filter disks (Whatman) and allowed to dry for 30 min. The filters were washed with 0.3 M ammonium formate, pH 8 (150 mL/wash until the cpm in 1 mL wash is less than 100, usually 6 washes). The filters were counted in 5-mL scintillation fluid in a scintillation counter.

The percentage of inhibition was calculated according to the following equation:

$$\%\text{Inhibition} = [1-(\text{cpm in test reaction} - \text{cpm in blank}) / (\text{cpm in control reaction} - \text{cpm in blank})] \times 100.$$

Representative compounds tested in the HCV NS5B polymerase assay exhibited $IC_{50}$'s less than 100 micromolar.

B. Assay for Inhibition of HCV RNA Replication:

The compounds of the present invention were also evaluated for their ability to affect the replication of Hepatitis

C Virus RNA in cultured hepatoma (HuH-7) cells containing a subgenomic HCV Replicon. The details of the assay are described below. This Replicon assay is a modification of that described in V. Lohmann, F. Korner, J-O. Koch, U. Herian, L. Theilmann, and R. Bartenschlager, "Replication of a Sub-genomic Hepatitis C Virus RNAs in a Hepatoma Cell Line," Science 285:110 (1999).

Protocol:

The assay was an *in situ* Ribonuclease protection, Scintillation Proximity based-plate assay (SPA). 10,000 - 40,000 cells were plated in 100-200 μL, of media containing 0.8mg/mL G418 in 96-well cytostar plates (Amersham). Compounds were added to cells at various concentrations up to 100 μM in 1% DMSO at time 0 to 18 h and then cultured for 24-96 h. Cells were fixed (20 min, 10% formalin), permeabilized (20 min, 0.25% Triton X-100/PBS) and hybridized (overnight, 50°C) with a single-stranded $^{33}P$ RNA probe complementary to the (+) strand NS5B (or other genes) contained in the RNA viral genome. Cells were washed, treated with RNAse, washed, heated to 65°C and counted in a Top-Count. Inhibition of replication was read as a decrease in counts per minute (cpm).

Human HuH-7 hepatoma cells, which were selected to contain a subgenomic replicon, carry a cytoplasmic RNA consisting of an HCV 5' non-translated region (NTR), a neomycin selectable marker, an EMCV IRES (internal ribosome entry site), and HCV non-structural proteins NS3 through NS5B, followed by the 3' NTR.

Representative compounds tested in the replication assay exhibited $EC_{50}$'s less than 100 micromolar.

C. Assay for Intracellular Metabolism:

The compounds of the present invention were also evaluated for their ability to enter a human hepatoma cell line and be converted intracellularly into the corresponding nucleoside 5'-mono-, di-, and triphosphates.

Two cell lines, HuH-7 and HBI10A, were used for intracellular metabolism studies of the compounds of the present invention. HuH-7 is a human hepatoma cell line, and HBI10A denotes a clonal line derived from HuH-7 cells that harbors the HCV bicistronic replicon. HuH-7 cells were plated in complete Dulbecco's modified Eagle's medium containing 10% fetal bovine serum and HBI10A cells in the same containing G418 (0.8 mg/mL) at 1.5 x 106 cells/60-mm dish such that cells were 80% confluent at the time of compound addition. Tritiated compound was incubated at 2 μM in the cell medium for 3 or 23 h. Cells were collected, washed with phosphate-buffered saline, and counted. The cells were then extracted in 70% methanol, 20 mM EDTA, 20 mM EGTA, and centrifuged. The lysate was dried, and radiolabeled nucleotides were analyzed using an ion-pair reverse phase (C-18) HPLC on a Waters Millenium system connected to an in-line β-RAM scintillation detector (IN/US Systems). The HPLC mobile phases consisted of (a)10 mM potassium phosphate with 2 mM tetrabutylammonium hydroxide and (b) 50% methanol containing 10 mM potassium phosphate with 2 mM tetrabutylammonium hydroxide. Peak identification was made by comparison of retention times to standards. Activity is expressed as picomoles of nucleotide detected in 106 HuH-7 or HBI10A cells.

The nucleoside aryl phosphoramidates of the present invention were also evaluated for cellular toxicity and anti-viral specificity in the counterscreens described below.

C. COUNTERSCREENS:

The ability of the nucleoside aryl phosphoramidates of the present invention to inhibit human DNA polymerases was measured in the following assays.

a. Inhibition of Human DNA Polymerases alpha and beta:

Reaction Conditions:

50 μL reaction volume

Reaction buffer components:

20 mM Tris-HCl, pH 7.5
200 μg/mL bovine serum albumin
100 mM KCl
2 mM β-mercaptoethanol
10 mM $MgCl_2$

1.6 μM dA, dG, dC, dTTP
α-$^{33}$P-dATP

Enzyme and template:

0.05 mg/mL gapped fish sperm DNA template
0.01 U/μL DNA polymerase α or β

Reparation of gapped fish sperm DNA template:

Add 5 μL 1M $MgCl_2$ to 500 μL activated fish sperm DNA (USB 70076);
Warm to 37°C and add 30 μL of 65 U/μL of exonuclease III (GibcoBRL 18013-011); Incubate 5 min at 37°C;
Terminate reaction by heating to 65 °C for 10 min;
Load 50-100 μL aliquots onto Bio-spin 6 chromatography columns (Bio-Rad 732-6002) equilibrated with 20 mM Tris-HCl, pH 7.5;
Elute by centrifugation at 1,000Xg for 4 min;
Pool eluate and measure absorbance at 260 nm to determine concentration.

The DNA template was diluted into an appropriate volume of 20 mM Tris-HCl, pH 7.5 and the enzyme was diluted into an appropriate volume of 20 mM Tris-HCl, containing 2 mM β-mercaptoethanol, and 100 mM KCl. Template and enzyme were pipetted into microcentrifuge tubes or a 96 well plate. Blank reactions excluding enzyme and control reactions excluding test compound were also prepared using enzyme dilution buffer and test compound solvent, respectively. The reaction was initiated with reaction buffer with components as listed above. The reaction was incubated for 1 hour at 37°C. The reaction was quenched by the addition of 20 μL 0.5M EDTA. 50 μL of the quenched reaction was spotted onto Whatman DE81 filter disks and air dried. The filter disks were repeatedly washed with 150 mL 0.3M ammonium formate, pH 8 until 1 mL of wash is < 100 cpm. The disks were washed twice with 150 mL absolute ethanol and once with 150 mL anhydrous ether, dried and counted in 5 mL scintillation fluid.

The percentage of inhibition was calculated according to the following equation: %

$$\text{inhibition} = [1\text{-}(\text{cpm in test reaction - cpm in blank})/(\text{cpm in control reaction - cpm in blank})] \times 100.$$

b. Inhibition of Human DNA Polymerase gamma :

The potential for inhibition of human DNA polymerase gamma was measured in reactions that included 0.5 ng/μL enzyme; 10 μM dATP, dGTP, dCTP, and TTP; 2 μCi/reaction [α$^{3}$P]-dATP, and 0.4 μg/μL, activated fish sperm DNA (purchased from US Biochemical) in a buffer containing 20 mM Tris pH8, 2 mM β-mercaptoethanol, 50 mM KCl, 10 mM MgCl2, and 0.1 1 μg/μL BSA. Reactions were allowed to proceed for 1 h at 37°C and were quenched by addition of 0.5 M EDTA to a final concentration of 142 mM. Product formation was quantified by anion exchange filter binding and scintillation counting. Compounds were tested at up to 50 μM.

The percentage of inhibition was calculated according to the following equation: %

$$\text{inhibition} = [1\text{-}(\text{cpm in test reaction - cpm in blank})/(\text{cpm in control reaction - cpm in blank})] \times 100.$$

The ability of the nucleoside aryl phosphoramidates of the present invention to inhibit HIV infectivity and HIV spread was measured in the following assays.

c. HIV Infectivity Assay

Assays were performed with a variant of HeLa Magi cells expressing both CXCR4 and CCR5 selected for low background β-galactosidase (β-gal) expression. Cells were infected for 48 h, and β-gal production from the integrated HIV-1 LTR promoter was quantified with a chemiluminescent substrate (Galactolight Plus, Tropix, Bedford, MA). Inhibitors

were titrated (in duplicate) in twofold serial dilutions starting at 100 μM; percent inhibition at each concentration was calculated in relation to the control infection.

d. Inhibition of HIV Spread

The ability of the compounds of the present invention to inhibit the spread of the human immunedeficiency virus (HIV) was measured by the method described in U.S. Patent No. 5,413,999 (May 9, 1995), and J.P.Vacca, et al., Proc. Natl. Acad. Sci., 91: 4096-4100 (1994), which are incorporated by reference herein in their entirety.

The nucleoside aryl phosphoramidates of the present invention were also screened for cytotoxicity against cultured hepatoma (HuH-7) cells containing a subgenomic HCV Replicon in an MTS cell-based assay as described in the assay below. The HuH-7 cell line is described in H. Nakabayashi, et al., Cancer Res., 42: 3858 (1982).

e. Cytotoxicity assay:

Cell cultures were prepared in appropriate media at concentrations of approximately 1.5 x $10^5$ cells/mL for suspension cultures in 3 day incubations and 5.0 x104 cells/mL for adherent cultures in 3 day incubations. 99 μL of cell culture was transferred to wells of a 96-well tissue culture treated plate, and 1 μL, of 100-times final concentration of the test compound in DMSO was added. The plates were incubated at 37°C and 5% $CO_2$ for a specified period of time. After the incubation period, 20 μL of CellTiter 96 Aqueous One Solution Cell Proliferation Assay reagent (MTS) (Promega) was added to each well and the plates were incubated at 37°C and 5% $CO_2$ for an additional period of time up to 3 h. The plates were agitated to mix well and absorbance at 490 nm was read using a plate reader. A standard curve of suspension culture cells was prepared with known cell numbers just prior to the addition of MTS reagent. Metabolically active cells reduce MTS to formazan. Formazan absorbs at 490 nm. The absorbance at 490 nm in the presence of compound was compared to absorbance in cells without any compound added.
Reference: Cory, A. H. et al., "Use of an aqueous soluble tetrazolium/formazan assay for cell growth assays in culture," Cancer Commun. 3: 207 (1991).

The following assays were employed to measure the activity of the compounds of the present invention against other RNA-dependent RNA viruses:

a. Determination of In Vitro Antiviral Activity of Compounds Against Rhinovirus (Cytopathic Effect Inhibition Assay):

Assay conditions are described in the article by Sidwell and Huffman, "Use of disposable microtissue culture plates for antiviral and interferon induction studies," Appl. Microbiol. 22: 797-801 (1971).

V iruses:

Rhinovirus type 2 (RV-2), strain HGP, was used with KB cells and media (0.1% $NaHCO_3$, no antibiotics) as stated in the Sidwell and Huffman reference. The virus, obtained from the ATCC, was from a throat swab of an adult male with a mild acute febrile upper respiratory illness.
Rhinovirus type 9 (RV-9), strain 211, and rhinovirus type 14 (RV-14), strain Tow, were also obtained from the American Type Culture Collection (ATCC) in Rockville, MD. RV-9 was from human throat washings and RV-14 was from a throat swab of a young adult with upper respiratory illness. Both of these viruses were used with HeLa Ohio-1 cells (Dr. Fred Hayden, Univ. of VA) which were human cervical epitheloid carcinoma cells. MEM (Eagle's minimum essential medium) with 5% Fetal Bovine serum (FBS) and 0.1% $NaHCO_3$ was used as the growth medium.
Antiviral test medium for all three virus types was MEM with 5% FBS, 0.1% $NaHCO_3$, 50 μg gentamicin/mL, and 10 mM $MgCl_2$.
2000 μg/mL was the highest concentration used to assay the compounds of the present invention. Virus was added to the assay plate approximately 5 min after the test compound. Proper controls were also run. Assay plates were incubated with humidified air and 5% $CO_2$ at 37°C. Cytotoxicity was monitored in the control cells microscopically for morphologic changes. Regression analysis of the virus CPE data and the toxicity control data gave the ED50 (50% effective dose) and CC50 (50% cytotoxic concentration). The selectivity index (SI) was calculated by the formula: SI = CC50 ÷ ED50.

b. Determination of In Vitro Antiviral Activity of Compounds Against Dengue, Banzi, and Yellow Fever (CPE Inhibition Assay)

Assay details are provided in the Sidwell and Huffman reference above.

Viruses:

Dengue virus type 2, New Guinea strain, was obtained from the Center for Disease Control. Two lines of African green monkey kidney cells were used to culture the virus (Vero) and to perform antiviral testing (MA-104). Both Yellow fever virus, 17D strain, prepared from infected mouse brain, and Banzi virus, H 336 strain, isolated from the serum of a febrile boy in South Africa, were obtained from ATCC. Vero cells were used with both of these viruses and for assay.

Cells and Media:

MA-104 cells (BioWhittaker, Inc., Walkersville, MD) and Vero cells (ATCC) were used in Medium 199 with 5% FBS and 0.1% $NaHCO_3$ and without antibiotics.
Assay medium for dengue, yellow fever, and Banzi viruses was MEM, 2% FBS, 0.18% $NaHCO_3$ and 50 $\mu$g gentamicin/mL:
Antiviral testing of the compounds of the present invention was performed according to the Sidwell and Huffman reference and similar to the above rhinovirus antiviral testing. Adequate cytopathic effect (CPE) readings were achieved after 5-6 days for each of these viruses.

c. Determination of In Vitro Antiviral Activity of Compounds Against West Nile Virus (CPE Inhibition Assay)

Assay details are provided in the Sidwell and Huffman reference cited above. West Nile virus, New York isolate derived from crow brain, was obtained from the Center for Disease Control. Vero cells were grown and used as described above. Test medium was MEM, 1% FBS, 0.1% $NaHCO_3$ and 50 $\mu$g gentamicin/mL.
Antiviral testing of the compounds of the present invention was performed following the methods of Sidwell and Huffman which are similar to those used to assay for rhinovirus activity. Adequate cytopathic effect (CPE) readings were achieved after 5-6 days.

d. Determination of In Vitro Antiviral Activity of Compounds Against rhino, yellow fever, dengue, Banzi, and West Nile Viruses (Neutral Red Uptake Assay)

After performing the CPE inhibition assays above, an additional cytopathic detection method was used which is described in "Microtiter Assay for Interferon: Microspectrophotometric Quantitation of Cytopathic Effect," Appl. Environ. Microbiol. 31: 35-38 (1976). A Model EL309 microplate reader (Bio-Tek Instruments Inc.) was used to read the assay plate. ED50's and CD50's were calculated as above.

EXAMPLE OF A PHARMACEUTICAL FORMULATION

As a specifit embodiment of an oral composition of a compound of the present invention, 50 mg of the compound of Example 1 or Example 2 is formulated withs sufficient finely divided lactose to provide total amount of 580 of 590 mg to fill a size O hard gelatin capsule.

## Claims

1. A compound of the structural formula I:

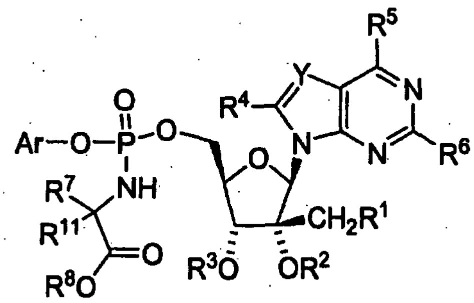

(I)

or a pharmaceutically acceptable salt thereof; wherein

Y is N;
Ar is phenyl unsubstituted or substituted with one to three substituents independently selected from the group consisting of halogen, $C_{1-4}$ alkyl, $C_{1-4}$ alcoxy, $C_{1-4}$ alkylthio, cyano, nitro, amino, carboxy, trifluoromethyl, $C_{1-4}$ alkylamino, di($C_{1-4}$ alkyl)amino, $C_{1-4}$ alkylcarbonyl, $C_{1-4}$ alkylcarbonyloxy, and $C_{1-4}$ alkyloxycarbonyl;
$R^1$ is selected from the group consisting of hydrogen, fluoro, azido, amino, hydroxy, $C_{1-3}$ alkoxy, mercapto, and $C_{1-3}$ alkylthio;
$R^2$ and $R^3$ are each independently selected from the group consisting of hydrogen, methyl, $C_{1-16}$ akylcarbonyl, $C_{2-18}$ alkenylcarbonyl, $C_{1-10}$ akyloxycarbonyl, $C_{3-6}$ cycloalkylcarbonyl, and $C_{36}$ cycloalkyloxycarbonyl;
R4 is hydrogen, halogen, methyl, azido, or amino;.
$R^5$ and $R^6$ are each independently selected from the group consisting of hydrogen, hydroxy, halogen, $C_{1-4}$ alkoxy, amino, $C_{1-4}$ alkylamino, di($C_{1-4}$ alkyl)amino, $C_{3-6}$ cycloalkylamino, di($C_{3-6}$ cycloalkyl)amino, benzylamino, dibenzylamino, or $C_{4-6}$ heterocycloatkyl, wherein alkyl,
cycloalkyl, benzyl, and heterocycloalkyl are unsubstituted or substituted with one to five groups independently selected from halogen, hydroxy, amino, $C_{1-4}$ alkyl, and $C_{1-4}$ alkoxy;
$R^7$ is hydrogen, $C_{1-5}$ alkyl, phenyl or benzyl;
wherein alkyl is unsubstituted or substituted with one substituent selected from the group consisting of hydroxy, methoxy, amino, carboxy, carbamoyl, guanidino, mercapto, methylthio, 1*H*-imidazolyl, and 1*H*-indol-3-yl; and wherein phenyl and benzyl are unsubstituted or substituted with one to two substituents independently selected from the group consisting of halogen, hydroxy, and methoxy;
$R^8$ is hydrogen, $C_{1-6}$ alkyl, $C_{3-6}$ cycloakyl, phenyl, or benzyl;
wherein alkyl and cycloalkyl are unsubstituted or substituted with one to three substituents independently selected from halogen, hydroxy, carboxy, $C_{1-4}$ alkoxy; and wherein phenyl and benzyl are unsubstituted or substituted with one to three substituents independently selected from halogen, hydroxy, cyano, $C_{1-4}$ alkoxy, and trifluoromethyl; and
$R^{11}$ is hydrogen or methyl.

2. The compound of Claim 1 wherein $R^1$ is hydrogen or fluoro, and $R^2$ and $R^3$ are hydrogen.

3. The compound of Claim 2 wherein $R^4$ is hydrogen.

4. The compound of any one of Claims 1 to 3 wherein Ar is unsubstituted phenyl.

5. The compound of any one of Claims 1 to 4 wherein $R^{11}$ is hydrogen and $R^7$ is selected from the group consisting of hydrogen, methyl, ethyl, n-propyl, isopropyl, isobutyl, 2-methyl-1-propyl, hydroxymethyl, mercaptomethyl, carboxymethyl, carbamoylmethyl, 1-hydroxyethyl, 2-carboxyethyl, 2-carbamoylethyl, 2-methylthioethyl, 4-amino-1-butyl, 3-amino,-1-propyl, 3-guanidino-1-propyl, 1*H*-imidazol-4-ylmethyl, phenyl, 4-hydroxybenzyl, and 1*H*-indol-3-ylmethyl.

6. The compound of Claim 5 wherein $R^7$ is methyl or benzyl.

7. The compound of any one of Claims 1 to 6 wherein $R^8$ is $C_{1-6}$ alkyl, cyclohexyl, phenyl or benzyl.

8. The compound of Claim 7 wherein $R^8$ is methyl.

9. The compound of Claim 1 wherein Ar is unsubstituted phenyl, $R^7$ is methyl or benzyl, $R^8$ is methyl, and $R^{11}$ is hydrogen.

10. The compound of Claim 9 which is selected from:

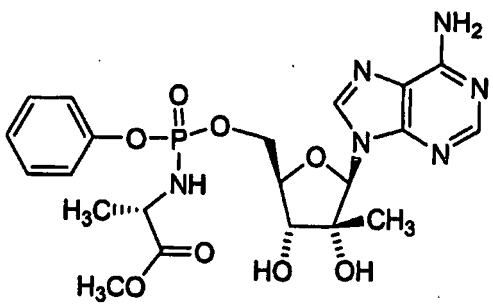

and

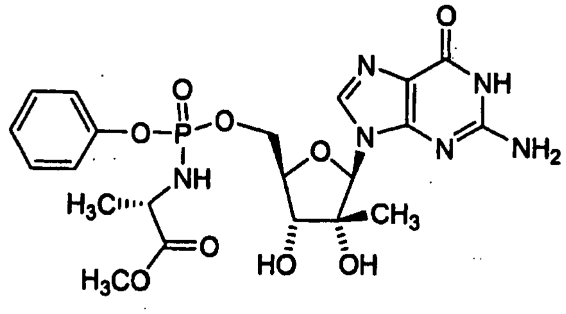

and pharmaceutically acceptable salts thereon

**11.** A pharmaceutical composition comprising a compound of any one of Claims 1 to 10 or a pharmaceutically acceptable salt thereof and a pharmaceutically acceptable carrier.

**12.** A compound of any one of Claims 1 to 10 or a pharmaceutically acceptable salt thereof for use in therapy.

**13.** Use of a compound of any one of claims 1 to 10 or a pharmaceutically acceptable salt thereof for in the manufacture of a medicament for the treatment of hepatitis C virus infection in a mammal.

**14.** A compound for use according to claim 12 wherein the use is a treatment of hepatitis C virus infection in a mammal.

**15.** A combination of a compound of any one of Claims 1 to 10 or a pharmaceutically acceptable salt thereof and another agent active against HCV.

**Patentansprüche**

**1.** Eine Verbindung der Strukturformel I:

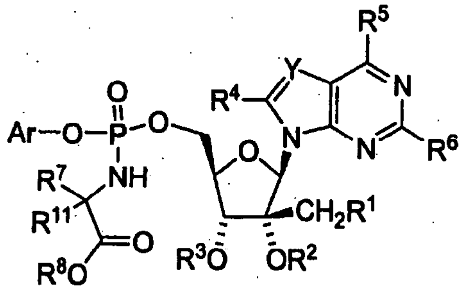

(I)

oder ein pharmazeutisch annehmbares Salz davon, wobei
Y N ist,
Ar Phenyl ist, unsubstituiert oder substituiert mit einem bis drei Substituenten, unabhängig ausgewählt aus der Gruppe, bestehend aus Halogen, $C_{1-4}$-Alkyl, $C_{1-4}$-Alkoxy, $C_{1-4}$-Alkylthio, Cyano, Nitro, Amino, Carboxy, Trifluormethyl, $C_{1-4}$-Alkylamino, Di($C_{1-4}$-alkyl)amino, $C_{1-4}$-Alkylcarbonyl, $C_{1-4}$-Alkylcarbonyloxy und $C_{1-4}$-Alkyloxycarbonyl,
$R^1$ ausgewählt ist aus der Gruppe, bestehend aus Wasserstoff, Fluor, Azido, Amino, Hydroxy, $C_{1-3}$-Alkoxy, Mercapto und $C_{1-3}$-Alkylthio,
$R^2$ und $R^3$ jeweils unabhängig ausgewählt sind aus der Gruppe, bestehend aus Wasserstoff, Methyl, $C_{1-16}$-Alkylcarbonyl, $C_{2-18}$-Alkenylcarbonyl, $C_{1-10}$-Alkyloxycarbonyl, $C_{3-6}$-Cycloalkylcarbonyl und $C_{3-6}$-Cycloalkyloxycarbonyl,
$R^4$ Wasserstoff, Halogen, Methyl, Azido oder Amino ist,

$R^5$ und $R^6$ jeweils unabhängig ausgewählt sind aus der Gruppe, bestehend aus Wasserstoff, Hydroxy, Halogen, $C_{1-4}$-Alkoxy, Amino, $C_{1-4}$-Alkylamino, Di($C_{1-4}$-alkyl)amino, $C_{3-6}$-Cycloalkylamino, Di($C_{3-6}$-cycloalkyl)amino, Benzylamino, Dibenzylamino oder $C_{4-6}$-Heterocycloalkyl, wobei Alkyl, Cycloalkyl, Benzyl und Heterocycloalkyl unsubstituiert oder substituiert sind mit einem bis fünf Gruppen, unabhängig ausgewählt aus Halogen, Hydroxy, Amino, $C_{1-4}$-Alkyl und $C_{1-4}$-Alkoxy,
$R^7$ Wasserstoff, $C_{1-5}$-Alkyl, Phenyl oder Benzyl ist,
wobei Alkyl unsubstituiert oder substituiert ist mit einem Substituenten, ausgewählt aus der Gruppe, bestehend aus Hydroxy, Methoxy, Amino, Carboxy, Carbamoyl, Guanidino, Mercapto, Methylthio, 1*H*-Imidazolyl und 1*H*-Indol-3-yl, und wobei Phenyl und Benzyl unsubstituiert oder substituiert sind mit einem bis zwei Substituenten, unabhängig ausgewählt aus der Gruppe, stehend aus Halogen, Hydroxy und Methoxy,
$R^8$ Wasserstoff, $C_{1-6}$-Alkyl, $C_{3-6}$-Cycloalkyl, Phenyl oder Benzyl ist,
wobei Alkyl und Cycloalkyl unsubstituiert oder substituiert sind mit einem bis drei Substituenten, unabhängig ausgewählt aus Halogen, Hydroxy, Carboxy, $C_{1-4}$-Alkoxy, und wobei Phenyl und Benzyl unsubstituiert oder substituiert sind mit einem bis drei Substituenten, unabhängig ausgewählt aus Halogen, Hydroxy, Cyano, $C_{1-4}$-Alkoxy und Trifluormethyl, und
$R^{11}$ Wasserstoff oder Methyl ist.

**2.** Die Verbindung nach Anspruch 1, wobei $R^1$ Wasserstoff oder Fluor ist und $R^2$ und $R^3$ Wasserstoff sind.

**3.** Die Verbindung nach Anspruch 2, wobei $R^4$ Wasserstoff ist.

**4.** Die Verbindung nach einem der Ansprüche 1 bis 3, wobei Ar unsubstituiertes Phenyl ist.

**5.** Die Verbindung nach einem der Ansprüche 1 bis 4, wobei $R^{11}$ Wasserstoff ist und $R^7$ ausgewählt ist aus der Gruppe, bestehend aus Wasserstoff, Methyl, Ethyl, n-Propyl, Isopropyl, Isobutyl, 2-Methyl-1-propyl, Hydroxymethyl, Mercaptomethyl, Carboxymethyl, Carbamoylmethyl, 1-Hydroxyethyl, 2-Carboxyethyl, 2-Carbamoylethyl, 2-Methylthioethyl, 4-Amino-1-butyl, 3-Amino-1-propyl, 3-Guanidino-1-propyl, 1*H*-Imidazol-4-ylmethyl, Phenyl, 4-Hydroxybenzyl und 1*H*-Indol-3-ylmethyl.

**6.** Die Verbindung nach Anspruch 5, wobei $R^7$ Methyl oder Benzyl ist.

**7.** Die Verbindung nach einem der Ansprüche 1 bis 6, wobei $R^8$ $C_{1-6}$-Alkyl, Cyclohexyl, Phenyl oder Benzyl ist.

**8.** Die Verbindung nach Anspruch 7, wobei $R^8$ Methyl ist.

**9.** Die Verbindung nach Anspruch 1, wobei Ar unsubstituiertes Phenyl ist, $R^7$ Methyl oder Benzyl ist, $R^8$ Methyl ist und $R^{11}$ Wasserstoff ist.

**10.** Die Verbindung nach Anspruch 9, die ausgewählt ist aus

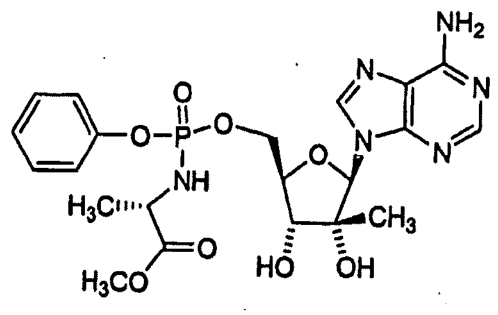

und

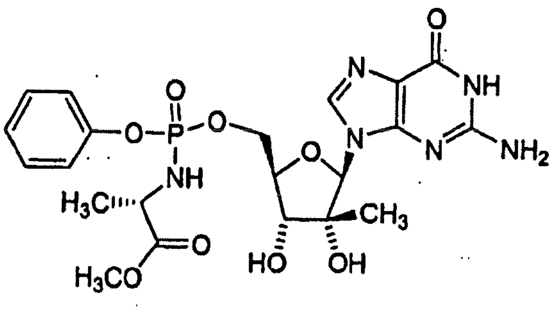

und pharmazeutisch annehmbare Salze davon.

11. Eine pharmazeutische Zusammensetzung, umfassend eine Verbindung nach einem der Ansprüche 1 bis 10 oder ein pharmazeutisch annehmbares Salz davon und einen pharmazeutisch annehmbaren Träger.

12. Eine Verbindung nach einem der Ansprüche 1 bis 10 oder ein pharmazeutisch annehmbares Salz davon zur Verwendung in der Therapie.

13. Verwendung einer Verbindung nach einem der Ansprüche 1 bis 10 oder eines pharmazeutisch annehmbaren Salzes davon bei der Herstellung eines Medikaments zur Behandlung einer Hepatitis-C-Virus-Infektion bei einem Säuger.

14. Eine Verbindung zur Verwendung gemäß Anspruch 12, wobei die Verwendung eine Behandlung einer Hepatitis-C-Virusinfektion bei einem Säuger ist.

15. Eine Kombination aus einer Verbindung nach einem der Ansprüche 1 bis 10 oder einem pharmazeutisch annehmbaren Salz davon und einem weiteren wirksamen Mittel gegen HCV.

## Revendications

1. Composé de la formule développée I:

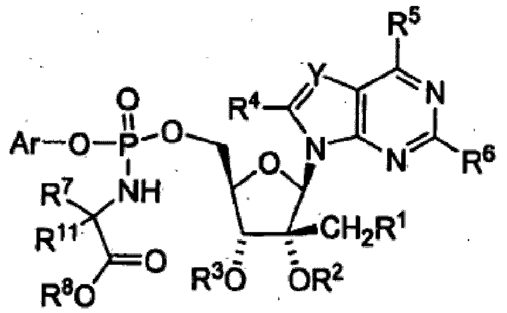

(I)

ou un sel pharmaceutiquement acceptable de celui-ci; dans lequel:

Y est N;

Ar est un phényle non substitué ou substitué par un à trois substituants sélectionnés indépendamment parmi le groupe consistant en halogène, alkyle $C_{1-4}$, alcoxy $C_{1-4}$, alkylthio $C_{1-4}$, cyano, nitro, amino, carboxy, trifluorométhyle, alkylamino $C_{1-4}$, di(alkyle $C_{1-4}$)amino, alkylcarbonyle $C_{1-4}$, alkylcarbonyloxy $C_{1-4}$ et alkyloxycarbonyle $C_{14}$;

$R^1$ est sélectionné parmi le groupe consistant en hydrogène, fluoro, azido, amino, hydroxy, alcoxy $C_{1-3}$, mercapto et alkylthio $C_{1-3}$;

$R^2$ et $R^3$ sont sélectionnés chacun indépendamment parmi le groupe consistant en hydrogène, méthyle, alkyl-

carbonyle $C_{1-16}$, alcénylcarbonyle $C_{2-18}$, alkyloxycarbonyle $C_{110}$, cycloalkylcarbonyle $C_{3-6}$ et cycloalkyloxycarbonyle $C_{3-6}$;

$R^4$ est un hydrogène, halogène, méthyle, azido ou amino;

$R^5$ et $R^6$ sont sélectionnés chacun indépendamment parmi le groupe consistant en hydrogène, hydroxy, halogène, alcoxy $C_{1-4}$, amino, alkylamino $C_{1-4}$, di(alkyle $C_{1-4}$)amino, cycloalkylamino $C_{3-6}$, di(cycloalkyle $C_{3-6}$)amino, benzylamino, dibenzylamino ou hétérocycloalkyle $C_{4-6}$ où l'alkyle, le cycloalkyle, le benzyle et l'hétérocycloalkyle sont non substitués ou substitués par un à cinq groupes sélectionnés indépendamment parmi halogène, hydroxy, amino, alkyle $C_{1-4}$ et alcoxy $C_{1-4}$;

$R^7$ est un hydrogène, alkyle $C_{1-5}$, phényle ou benzyle;

où l'alkyle est non substitué ou substitué par un substituant sélectionné parmi le groupe consistant en hydroxy, méthoxy, amino, carboxy, carbamoyle, guanidino, mercapto, méthylthio, 1H-imidazolyle et 1H-indol-3-yle, et où le phényle et le benzyle sont non substitués ou substitués par un à deux substituants sélectionnés indépendamment parmi le groupe consistant en halogène, hydroxy et méthoxy;

$R^8$ est un hydrogène, alkyle $C_{1-6}$, cycloalkyle $C_{3-6}$, phényle ou benzyle;

où l'alkyle et le cycloalkyle sont non substitués ou substitués par un à trois substituants sélectionnés indépendamment parmi halogène, hydroxy, carboxy, alcoxy $C_{14}$; et où le phényle et le benzyle sont non substitués ou substitués par un à trois substituants sélectionnés indépendamment parmi halogène, hydroxy, cyano, alcoxy $C_{1-4}$ et trifluorométhyle; et

$R^{11}$ est un hydrogène ou un méthyle.

**2.** Composé selon la revendication 1, dans lequel $R^1$ est un hydrogène ou un fluoro et $R^2$ et $R^3$ sont un hydrogène.

**3.** Composé selon la revendication 2, dans lequel $R^4$ est un hydrogène.

**4.** Composé selon l'une quelconque des revendications 1 à 3, dans lequel Ar est un phényle non substitué.

**5.** Composé selon l'une quelconque des revendications 1 à 4, dans lequel $R^{11}$ est un hydrogène et $R^7$ est sélectionné parmi le groupe consistant en hydrogène, méthyle, éthyle, n-propyle, isopropyle, isobutyle, 2-méthyl-1-propyle, hydroxyméthyle, mercaptométhyle, carboxyméthyle, carbamoylméthyle, 1-hydroxyéthyle, 2-carboxyéthyle, 2-carbamoyléthyle, 2-méthylthioéthyle, 4-amino-1-butyle, 3-amino-1-propyle, 3-guanidino-1-propyle, 1H-imidazol-4-ylméthyle, phényle, 4-hydroxybenzyle et 1H-indol-3-ylméthyle.

**6.** Composé selon la revendication 5, dans lequel $R^7$ est un méthyle ou un benzyle.

**7.** Composé selon l'une quelconque des revendications 1 à 6, dans lequel $R^8$ est un alkyle $C_{1-6}$, cyclohexyle, phényle ou benzyle.

**8.** Composé selon la revendication 7, dans lequel $R^8$ est un méthyle.

**9.** Composé selon la revendication 1, dans lequel Ar est un phényle non substitué, $R^7$ est un méthyle ou un benzyle, $R^8$ est un méthyle et $R^{11}$ est un hydrogène.

**10.** Composé selon la revendication 9, qui est sélectionné parmi

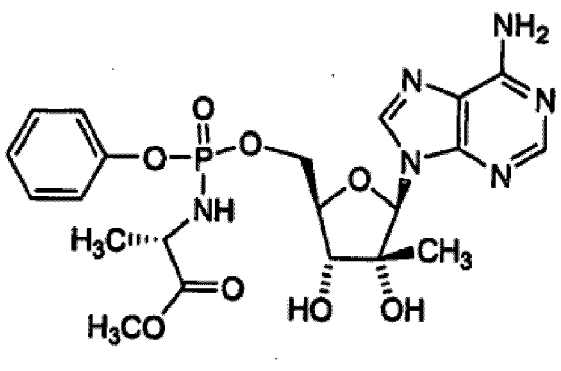

et

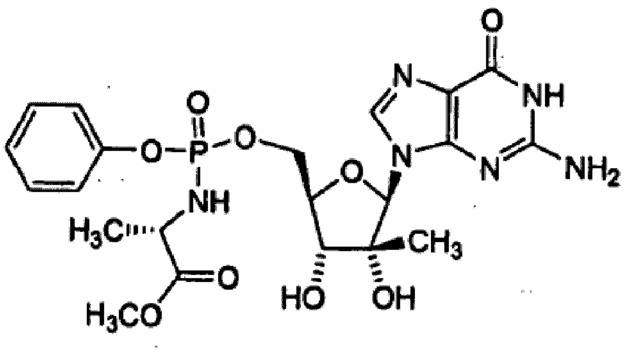

et des sels pharmaceutiquement acceptables de ceux-ci.

**11.** Composition pharmaceutique comprenant un composé selon l'une quelconque des revendications 1 à 10 ou un sel pharmaceutiquement acceptable de celui-ci et un véhicule pharmaceutiquement acceptable.

**12.** Composé selon l'une quelconque des revendications 1 à 10 ou un sel pharmaceutiquement acceptable de celui-ci à utiliser en thérapie.

**13.** Utilisation d'un composé selon l'une quelconque des revendications 1 à 10 ou d'un sel pharmaceutiquement acceptable de celui-ci dans la fabrication d'un médicament pour le traitement d'une infection par le virus de l'hépatite C chez un mammifère.

**14.** Composé à utiliser selon la revendication 12, où l'utilisation est le traitement d'une infection par le virus de l'hépatite C chez un mammifère.

**15.** Combinaison d'un composé selon l'une quelconque des revendications 1 à 10 ou d'un sel pharmaceutiquement acceptable de celui-ci et d'un autre agent actif contre le VHC.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02057425 A
- WO 0160379 A
- WO 9822496 A
- WO 9846630 A
- WO 9907733 A
- WO 9907734 A
- WO 9938888 A
- WO 9950230 A
- WO 9964442 A
- WO 0009543 A
- WO 0059929 A
- GB 2337262 A
- WO 0248116 A
- WO 0248172 A
- US 6323180 B
- WO 9741211 A
- WO 0100622 A
- WO 0025780 A
- US 3480613 A
- WO 0190121 A
- WO 0192282 A
- WO 0232920 A
- WO 04002999 A
- WO 04003000 A
- WO 04002422 A
- WO 0251425 A
- WO 0179246 A
- WO 0248165 A
- WO 0168663 A
- WO 9943691 A
- WO 0218404 A
- US 20020019363 A
- WO 02100415 A
- WO 03026589 A
- WO 03026675 A
- WO 03093290 A
- US 20030236216 A
- US 20040006007 A
- WO 04011478 A
- WO 04013300 A
- US 20040063658 A
- WO 04028481 A
- WO 0177091 A
- WO 0147883 A
- WO 0204425 A
- WO 0206246 A
- WO 0220497 A
- WO 02057287 A
- US 6455513 B
- US 5413999 A

### Non-patent literature cited in the description

- **B. DYMOCK et al.** Novel approaches to the treatment of hepatitis C virus infection. *Antiviral Chemistry & Chemotherapy,* 2000, vol. 11, 79-96
- **H. ROSEN et al.** Hepatitis C virus: current understanding and prospects for future therapies. *Molecular Medicine Today,* 1999, vol. 5, 393-399
- **D. MORADPOUR et al.** Current and evolving therapies for hepatitis C. *European J. Gastroenterol. Hepatol.,* 1999, vol. 11, 1189-1202
- **R. BARTENSCHLAGER.** Candidate Targets for Hepatitis C Virus-Specific Antiviral Therapy. *Intervirology,* 1997, vol. 40, 378-393
- **G.M. LAUER ; B.D. WALKER.** Hepatitis C Virus Infection. *N. Engl. J. Med.,* 2001, vol. 345, 41-52
- **B.W. DYMOCK.** Emerging therapies for hepatitis C virus infection. *Emerging Drugs,* 2001, vol. 6, 13-42
- **C. CRABB.** Hard-Won Advances Spark Excitement about Hepatitis C. *Science,* 2001, 506-507
- **K. ISHI et al.** Expression of Hepatitis C Virus NS5B Protein: Characterization of Its RNA Polymerase Activity and RNA Binding. *Hepatology,* 1999, vol. 29, 1227-1235
- **V. LOHMANN et al.** Biochemical and Kinetic Analyses of NS5B RNA-Dependent RNA Polymerase of the Hepatitis C Virus. *Virology,* 1998, vol. 249, 108-118
- **M.P. WALKER et al.** Promising candidates for the treatment of chronic hepatitis C. *Expert Opin. Invest. Drugs,* 2003, vol. 12, 1269-1280
- **P. HOFFMANN et al.** Recent patents on experimental therapy for hepatitis C virus infection (1999-2002). *Expert Opin. Ther. Patents,* 2003, vol. 13, 1707-1723
- **A.E. ELDRUP et al.** Structure-Activity Relationship of Purine Ribonucleosides for Inhibition of HCV RNA-Dependent RNA Polymerase. *J. Med. Chem,* 2004, vol. 47, 2283-2295

- **A.C. ALLISON ; E.M. EUGUI.** *Agents Action,* 1993, vol. 44, 165
- **J. KIRSCHBAUM.** *Anal. Profiles Drug Subs.,* 1983, vol. 12, 1-36
- **R. E. HARRY-O'KURU et al.** *J. Org. Chem.,* 1997, vol. 62, 1754-1759
- **M. S. WOLFE et al.** *Tetrahedron Lett.,* 1995, vol. 36, 7611-7614
- **J.O. SAUNDERS ; S.A. RAYBUCK.** Inosine Monophosphate Dehydrogenase: Consideration of Structure, Kinetics, and Therapeutic Potential. *Ann. Rep. Med. Chem.,* 2000, vol. 35, 201-210
- Design of Prodrugs. Elsevier, 1985
- **C. MCGUIGAN et al.** *J. Med. Chem.,* 1993, vol. 36, 1048
- *J. Med. Chem.,* 1993, vol. 36, 1048
- **V. LOHMANN ; F. KORNER ; J-O. KOCH ; U. HERIAN ; L. THEILMANN ; R. BARTENSCHLAGER.** Replication of a Sub-genomic Hepatitis C Virus RNAs in a Hepatoma Cell Line. *Science,* 1999, vol. 285, 110
- **J.P.VACCA et al.** *Proc. Natl. Acad. Sci.,* 1994, vol. 91, 4096-4100
- **H. NAKABAYASHI et al.** *Cancer Res.,* 1982, vol. 42, 3858
- **CORY, A. H. et al.** Use of an aqueous soluble tetrazolium/formazan assay for cell growth assays in culture. *Cancer Commun.,* 1991, vol. 3, 207
- **SIDWELL ; HUFFMAN.** Use of disposable microtissue culture plates for antiviral and interferon induction studies. *Appl. Microbiol.,* 1971, vol. 22, 797-801
- Microtiter Assay for Interferon: Microspectrophotometric Quantitation of Cytopathic Effect. *Appl. Environ. Microbiol.,* 1976, vol. 31, 35-38